# EUROPEAN PATENT APPLICATION

(11) **EP 1 312 607 A1**
(43) Date of publication of application: **21.05.2003**
(21) Application number: 01127296.0
(22) Date of filing: 17.11.2001
(51) Int. Cl.: C07D 239/46, C07D 239/56, C07D 213/71, C07C 333/20, A61K 31/506, A61P 31/04

(54) **Dithiocarbamate derivatives and their use as antibacterial agents**

(71) Applicant: MEDAC GmbH, 22880 Wedel (DE)
(72) Inventor: Makarov, Vadim, Dr., Moscow 121059 (RU); Möllmann, Ute, Dr., 07749 Jena (DE)
(74) Representative: Weber-Quitzau, Martin, Dr.

(57) **Abstract**

Compounds of the formula I 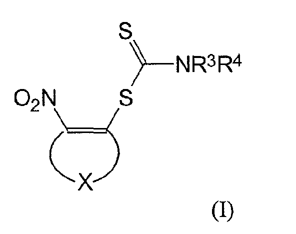 wherein X is a bivalent residue selected from the group consisting of 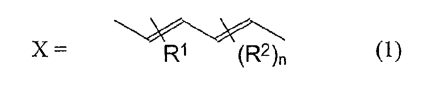 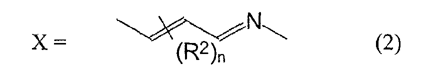 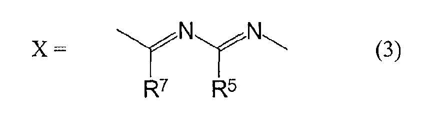 have excellent antibacterial activities and are useful agents for the therapeutic or prophylactic treatment of infectious diseases in mammals (humans and animals) caused by bacteria, especially diseases like tuberculosis (TB) and lepra caused by mycobacteria and infectious diseases caused by staphylococci.

## Description

The invention relates to novel dithiocarbamate derivatives and their use as antibacterial agents in infectious diseases of mammals (humans and animals) caused by bacteria, especially diseases like tuberculosis (TB) and lepra caused by mycobacteria and infectious diseases caused by staphylococci.

As known, there is a threadful worldwide increase in tuberculosis infections with mycobacteria which developed resistance against the available therapeutics (B.R.Bloom, J.L.Murray, tuberculosis: commentary on a reemergent killer. Science 257, 1992, 1055-1064). Extremely dangerous is the development of multidrug resistant (MDR) mycobacteria. These are mycobacteria, resistant at least against two of the most active tuberculosis drugs, isoniazid and rifampicin, but also against streptomycin, pyranzinamid and ethambutol. The proportion of MDR-TB in some countries is already more than 20%. In Germany resistance against a single tuberculosis drug raised since 1996 for 50%. Together with the increased number of diseases generally, worldwide tuberculosis causes a number of 3.000.000 deaths annually. Another growing problem is the therapy of infections with multiresistant staphylococci (M. Kresken, Bundesgesundheitsblatt 38,1996, 170-178).

For the treatment of such diseases there is an urgent need for new drugs with new mechanisms of actions, especially to overcome drug resistance and to overcome the known dramatic side effects of the available drugs.

The present invention aims at the generation of new compounds with activity against mycobacteria as potential new tuberculosis drugs and with activity against other Gram-positive pathogens like staphylococci to overcome problems concerning resistance and drug intolerance.

This aim has been solved by providing compounds of the formula I 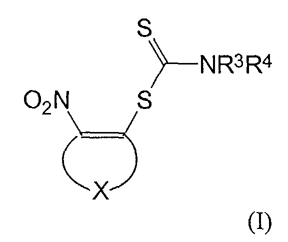 wherein X is a bivalent residue selected from the group consisting of 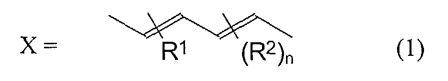 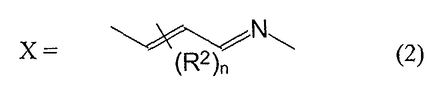 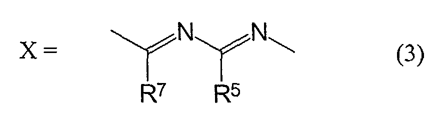 wherein
R¹ is CN, CONR³R⁴, CONHNR³R⁴;
R² is H, NO₂, CN, CONR³R⁴, COOR⁵, CHO, halogen or a saturated or unsaturated, linear or branched aliphatic radical having 1-7 chain members, OR⁵, SR⁵, NR³R⁴, trifluoromethyl, phenyl;
n is 0-3;
R³ and R⁴ are, independently from each other, H, a saturated or unsaturated, linear or branched aliphatic radical having 1-7 chain members, cycloalkyl having 3-6 carbon atoms, benzyl, naphthyl, OR⁵; or NR³R⁴ is morpholino; or R³ and R⁴ together represent a bivalent radical -(CH₂)ₘ- wherein m is 2-7, or a bivalent radical -(CH₂CH₂)NR⁶(CH₂CH₂)-, or R³ and R⁴ together represent a bivalent radical 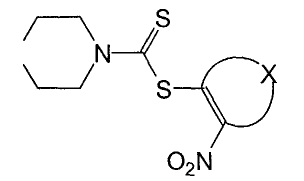 R⁵ is H or a saturated or unsaturated, linear or branched aliphatic radical having 1-7 chain members, trifluoromethyl, benzyl or phenyl;
R⁶ is H or a saturated or unsaturated, linear or branched aliphatic radical having 1-7 chain members, trifluoromethyl, benzyl or phenyl;
R⁷ is H, halogen or a saturated or unsaturated, linear or branched aliphatic radical having 1-7 chain members, OR⁵, SR⁵, NR³R⁴, trifluoromethyl, phenyl;
with the exclusion of the known compounds
1,5-dinitro-3-cyano-6-dimethyldithiocarbamoyl-benzene (J.Org. Chem., 1979, 44(2), p.267-274), 3,5-dinitro-2-dimethyldithiocarbamoylpyridine, 3,5-dinitro-6-dimethylamino-2-dimethyl-dithiocarbamoyl-pyridine, 4-methoxy-6-diethyldithiocarbamoyl-5-nitro-pyrimidine, 4-methoxy-6-dipropyldithiocarbamoyl-5-nitro-pyrimidine, 4-dimethylamino-6-diethyldithiocarbamoyl-5-nitro-pyrimidine, 4-dimethylamino-6-dipropyldithiocarbamoyl-5-nitro-pyrimidine (Khim. Geterotsikl. Soedin., (Rus.) 1994, 10, p. 1420-1423, CA 122:314512), and 4-methylamino-6-diethyldithiocarbamoyl-5-nitro-pyrimidine (Acta Cryst. Section C, 2001, C57, p.72-75).

In a preferred embodiment the invention concerns compounds of the formula (I) selected from the group consisting of
3,5-dinitro-2-R³R⁴-dithiocarbamoyl-benzonitriles,
3,5-di-nitro-2-R³R⁴-dithiocarbamoyl-benzamides,
3,5-dinitro-2-R³R⁴-dithiocarbamoyl-pyridines, and
4-R⁷-2-R⁵-6-R³R⁴-dithiocarbamoyl-5-nitro-pyrimidines,
wherein R³, R⁴, R⁵ and R⁷ have the above meanings,
except 3,5-dinitro-2-dimethyl-dithiocarbamoyl-pyridine, 4-methoxy-6-diethyldithiocarbamoyl-5-nitro-pyrimidine, 4-methoxy-6-dipropyldithiocarbamoyl-5-nitro-pyrimidine, 4-dimethylamino-6-diethyldithiocarbamoyl-5-nitro-pyrimidine, 4-dimethylamino-6-dipropyl-dithiocarbamoyl-5-nitro-pyrimidine and 4-methylamino-6-diethyldithiocarbamoyl-5-nitro-pyrimidine.

The present invention is even more particularly concerned with at least one compound selected from the group consisting of
4-isopropylamino-6-diethyldithiocarbamoyl-5-nitropyrimidine,
4-cyclopropylamino-6-diethyldithiocarbamoyl-5-nitropyrimidine,
3,5-dinitro-2-diethyldithiocarbamoylbenzonitril,
4-methylhydroxyamino-6-diethyldithiocarbamoyl-5-nitropyrimidine,
4-ethoxy-2-methyl-6-diethyldithiocarbamoyl-5-nitropyrimidine,
4-methylamino-6-dimethyldithiocarbamoyl-5-nitropyrimidine,
4-dimethylamino-6-dimethyldithiocarbamoyl-5-nitropyrimidine,
3,5-dinitro-2-dimethyldithiocarbamoylbenzonitrile,
4-ethoxy-2-methyl-6-tetramethylenedithiocarbamoyl-5-nitropyrimidine,
4-propoxy-6-dimethyldithiocarbamoyl-5-nitropyrimidine,
4-methylamino-6-tetramethylenedithiocarbamoyl-5-nitropyrimidine,
4-dimethylamino-6-tetramethylenedithiocarbamoyl-5-nitropyrimidine,
4-methoxy-6-dimethyldithiocarbamoyl-5-nitropyrimidine,
4-methoxy-6-tetramethylenedithiocarbamoyl-5-nitropyrimidine,
3,5-dinitro-2-hexamethylenedithiocarbamoylbenzamide,
3,5-dinitro-2-tetramethylenedithiocarbamoylbenzamide,
3,5-dinitro-2-tetramethylenedithiocarbamoylpyridine,
3,5-dinitro-2-hexamethylenedithiocarbamoylbenzonitril, and
4-methylamino-6-heptamethylenedithiocarbamoyl-5-nitropyrimidine.

The substituents mentioned hereinbefore or hereinafter have the following meanings: A saturated or unsaturated, linear or branched aliphatic radical having 1-7 chain members is, for example, a corresponding alkyl, alkenyl or alkinyl radical having 1-6 carbon atoms optionally interrupted by one oxygen or sulphur atom or by NR⁶. Compounds of the formula I wherein R³ and R⁴ together represent a bivalent radical 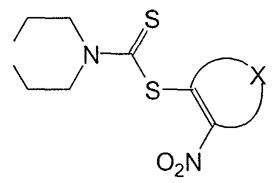 are preferably symmetrical with respect to the symmetric axis dividing the central piperazine ring into equal halves.

Surprisingly the compounds of the invention exhibit strong antibacterial activity, especially against mycobacteria with minimal inhibitory concentrations (MIC) in the range of 0,1-50 µg/ml for fast growing mycobacteria and of 3,12-12,5 µg/ml for M. *tuberculosis*, and against multiresistant staphylococci (MRSA) in a MIC range of. 0,4-50 µg/ml.

Thus, the compounds of the invention are useful for the treatment of bacterial infections, especially tuberculosis and other mycobacterial infections, in humans and in animals.

Accordingly, the invention concerns pharmaceutical compositions comprising a compound of the formula I.

The invention relates furthermore to a compound of the formula I for use in a method for the treatment of bacterial infections in mammals. Preferred compounds of the formula I for use in such method are those specifically listed above plus additionally 4-methylamino-6-diethyldithiocarbamoyl-5-nitropyrimidine.

The compounds of the invention are formulated for use by preparing a dilute solution or suspension in pharmacautically acceptable aqueous, organic or aqueous-organic medium for topical or parenteral administration by intravenous, subcutaneous or intramuscular injection, or for intranasal application; or are prepared in tablet, capsule or aqueous suspension form with conventional excipients for oral administration or as suppositorium.
The compounds can be used in dosages from 0,1 - 1000 mg/kg body weight..

The examples which follow in the subsequent experimental part serve to illustrate the invention but should not be construed as a limitation thereof.

The structures of the compounds of the invention were established by modes of synthesis and elementary analysis, and by nuclear magnetic resonance and/or mass spectra, X-ray analysis.

### Experimental Part

### Starting materials

Starting 4-chloro-6-R-5-nitropyrimidines were synthesized according to Chesney J.D., Gonzales-Sierra M., Pharm. Res., 1985, p.145-147 and Clark J., Parvizi B., Colmam R., J.Chem.Soc., Perkin Trans 1, 1976, p. 1004-1007; 3,5-dinitro-2-chlorobenzonitrile and 3,5-dinitro-2-chlorobenzamide were synthesized according to Thiel W., Mayer R., J.Prakt. Chemie, B328, 1986, p.497-514.

### Example 1

### 4-isopropylamino-6-diethyldithiocarbamoyl-5-nitropyrimidine (1)

A mixture of 4-isopropylamino-6-chloro-5-nitropyrimidine (0.94 g, 4.3 mmol), sodium diethyldithiocarbamate trihydrate (1.05 g, 4.6 mmol) and ethanol (25 mL) was mixed for 24 hours at room temperature. Reaction mixture was diluted by water (50 mL), yellow solid was filtered and purified by crystallization from ethanol to give 0.8 g of pure title compound as an yellow crystalline solid, m.p. 165-167°C.

| | | | | |
|---|---|---|---|---|
| Anal. Calcd. for C₁₂H₁₇N₅O₂S₂ | C, 44.02; | H, 5.23; | N, 21.39; | S, 19.59 |
| Found | C, 44.31; | H, 5.17; | N, 21.52; | S, 19.78 |

### Example 2

### 4-cyclopropylamino-6-diethyldithiocarbamoyl-5-nitropyrimidine (2)

Following the procedure of Example 1. Yellow crystalline solid, m.p. 143-145°C.

| | | | | |
|---|---|---|---|---|
| Anal. Calcd. for C₁₂H₁₇N₅O₂S₂ | C, 44.02; | H, 5.23; | N, 21.39; | S, 19.59 |
| Found | C, 44.17; | H, 5.31; | N, 21.39; | S, 19.38 |

### Example 3

### 3,5-dinitro-2-diethyldithiocarbamoylbenzonitril (3)

A mixture of 3,5-dinitro-2-chlorobenzonitrile (1.5 g, 6.6 mmol), sodium diethyldithiocarbamate trihydrate (1.6 g, 7.1 mmol) and ethanol (40 mL) was mixed for 2 hours at room temperature. Reaction mixture was treated by active charcoal, filtered off. Mother liquid was diluted by water (80 mL), yellow solid was filtered and purified by consecutive crystallization from *i*-propanol and acetone/water to give 1.2 g of pure title compound as an light yellow crystalline solid, m.p. 151-153°C.

| | | | | |
|---|---|---|---|---|
| Anal. Calcd. for C₁₂H₁₂N₄O₄S₂ | C, 42.34; | H, 3.55; | N, 16.46; | S, 18.84 |
| Found | C, 42.32; | H, 3.46; | N, 16.41; | S, 18.69 |

### Example 4

### 4-methylamino-6-dimethyldithiocarbamoyl-5-nitropyrimidine (7)

A mixture of 4-methylamino-6-chloro-5-nitropyrimidine (0.7 g, 3.7 mmol), sodium dimethyldithiocarbamate dihydrate (0.7 g, 3.9 mmol), ethanol (15 mL) and acetone (15 mL) was mixed for 4 hours at room temperature. Reaction mixture was diluted by water (60 mL), yellow solid was filtered and purified by crystallization from ethanol to give 0.5 g of pure title compound as an yellow crystalline solid, m.p. 136-137°C.

| | | | | |
|---|---|---|---|---|
| Anal. Calcd. for C₈H₁₁N₅O₂S₂ | C, 35.15; | H, 4.06; | N, 25.62; | S, 23.46 |
| Found | C, 34.95; | H, 4.11; | N, 25.53; | S, 23.58 |

### Example 5

### 4-methylamino-6-heptamethylenedithiocarbamoyl-5-nitropyrimidine (20)

Following the procedure of Example 4. Yellow crystalline solid, m.p. 180-182°C.

| | | | | |
|---|---|---|---|---|
| Anal. Calcd. for C₁₁H₁₅N₅O₂S₂ | C, 42.16; | H, 4.82; | N, 22.35; | S, 20.46 |
| Found | C, 42.11; | H, 5.01; | N, 22.42; | S, 20.49 |

### Example 6

### 4-methylamino-6-tetramethylenedithiocarbamoyl-5-nitropyrimidine (12)

Following the procedure of Example 4. Yellow crystalline solid, m.p. 213-215°C.

| | | | | |
|---|---|---|---|---|
| Anal. Calcd. for C₁₀H₁₃N₅O₂S₂: | C, 40.12; | H, 4.38; | N, 23.39; | S, 21.42 |
| Found: | C, 40.23; | H, 4.37; | N, 23.47; | S, 21.35 |

### Example 7

### 4-methylhydroxyamino-6-diethyldithiocarbamoyl-5-nitropyrimidine (5)

Following the procedure of Example 4. Yellow crystalline solid, m.p.92-94°C.

| | | | | |
|---|---|---|---|---|
| Anal. Calcd. for C₁₀H₁₅N₅O₃S₂ | C, 37.84; | H, 4.76; | N, 22.07; | S, 20.21 |
| Found | C, 37.81; | H, 4.57; | N, 22.01; | S, 20.33 |

### Example 8

### 4-dimethylamino-6-dimethyldithiocarbamoyl-5-nitropyrimidine (8)

Following the procedure of Example 4. Yellow crystalline solid, m.p.135-137°C.

| | | | | |
|---|---|---|---|---|
| Anal. Calcd. for C₉H₁₃N₅O₂S₂ | C, 37.62; | H, 4.56; | N, 24.37; | S, 22.32 |
| Found | C, 37.66; | H, 4.48; | N, 24.49; | S, 22.39 |

### Example 9

### 4-dimethylamino-6-tetramethylenedithiocarbamoyl-5-nitropyrimidine (13)

Following the procedure of Example 4. Yellow crystalline solid, m.p. 130-132°C.

| | | | | |
|---|---|---|---|---|
| Anal. Calcd. for C₁₁H₁₅N₅O₂S₂ | C, 42.16; | H, 4.82; | N, 22.35; | S, 20.46 |
| Found | C, 42.23; | H, 4.88; | N, 22.36; | S, 20.23 |

### Example 10

### 3,5-dinitro-2-dimethyldithiocarbamoylbenzonitrile (9)

A mixture of 3,5-dinitro-2-chlorobenzonitrile (2.0 g, 8.8 mmol), sodium dimethyldithiocarbamate dihydrate (1.7 g, 9.5 mmol) and ethanol (50 mL) was mixed for 0,5 hour at room temperature. Reaction mixture was diluted by water (100 mL), yellow solid was filtered and purified by consecutive crystallization from ethanol with addition of active charcoal to give 1.4 g of pure title compound as an orange solid, m.p. 147-149°C.

| | | | | |
|---|---|---|---|---|
| Anal. Calcd. for C₁₀H₈N₄O₄S₂ | C, 38.46; | H, 2.58; | N, 17.94; | S, 20.53 |
| Found | C, 38.61; | H, 2.58; | N, 17.93; | S, 20.52 |

### Example 11

### 3,5-dinitro-2-hexamethylenedithiocarbamoylbenzonitrile (19)

Following the procedure of Example 10. Yellow crystalline solid, m.p. 148-150°C.

| | | | | |
|---|---|---|---|---|
| Anal. Calcd. for C₁₄H₁₄N₄O₄S₂ | C, 45.62; | H, 3.85; | N, 15.29; | S, 17.50 |
| Found | C, 45.56; | H, 3.87; | N, 15.37; | S, 17.42 |

### Example 12

### 4-methoxy-6-dimethyldithiocarbamoyl-5-nitropyrimidine (14)

A solution of 4-methoxy-6-chloro-5-nitropyrimidine (0.5 g, 2.6 mmol), sodium dimethyldithiocarbamate dihydrate (0.6 g, 3.3 mmol) and ethanol (40 mL) was mixed for 1 hour at room temperature. Reaction mixture was diluted by water (80 mL), light yellow solid was filtered and purified by crystallization from methanol to give 0.4 g of pure title compound as an bright yellow crystalline solid, m.p. 122-125°C.

| | | | | |
|---|---|---|---|---|
| Anal. Calcd. for C₈H₁₀N₄O₃S₂: | C, 35.03; | H, 3.67; | N, 20.42; | S, 23.38 |
| Found: | C, 34.93; | H, 4.02; | N, 20.50; | S, 23.42 |

### Example 13

### 4-methoxy-6-tetramethylenedithiocarbamoyl-5-nitropyrimidine (15)

Following the procedure of Example 12. Yellow crystalline solid, m.p. 147-149°C.

| | | | | |
|---|---|---|---|---|
| Anal. Calcd. for C₁₀H₁₂N₄O₃S₂ | C, 39.99; | H, 4.03; | N, 18.65; | S, 21.35 |
| Found | C, 39.98; | H, 4.06; | N, 18.73; | S, 21.32 |

### Example 14

### 4-ethoxy-2-methyl-6-diethyldithiocarbamoyl-5-nitropyrimidine (6)

Following the procedure of Example 12. Yellow crystalline solid, m.p. 108-110°C.

| | | | | |
|---|---|---|---|---|
| Anal. Calcd. for C₁₂H₁₈N₄O₃S₂ | C, 43.62; | H, 5.49; | N, 16.96; | S, 19.41 |
| Found | C, 43.74; | H, 5.45; | N, 16.82; | S, 20.01 |

### Example 15

### 4-ethoxy-2-methyl-6-tetramethylenedithiocarbamoyl-5-nitropyrimidine (10)

Following the procedure of Example 12. Yellow crystalline solid, m.p. 100-102°C.

| | | | | |
|---|---|---|---|---|
| Anal. Calcd. for C₁₀H₁₃N₅O₂S₂: | C, 40.12; | H, 4.38; | N, 23.39; | S, 21.42 |
| Found: | C, 40.23; | H, 4.37; | N, 23.47; | S, 21.35 |

### Example 16

### 4-propoxy-6-dimethyldithiocarbamoyl-5-nitropyrimidine (11)

Following the procedure of Example 12. Yellow crystalline solid, m.p. 107-109°C.

| | | | | |
|---|---|---|---|---|
| Anal. Calcd. for C₁₀H₁₄N₄O₃S₂: | C, 39.72; | H, 4.67; | N, 18.53; | S, 21.21 |
| Found: | C, 40.04; | H, 4.65; | N, 18.42; | S, 21.56 |

### Example 17

### 3,5-dinitro-2-hexamethylenedithiocarbamoylbenzamide (16)

A solution of 3,5-dinitro-2-chlorobenzamide (0.7 g, 2.9 mmol), sodium hexamethylenedithiocarbamate dihydrate (0.7 g, 3.0 mmol) and ethanol (50 mL) was mixed for 0,5 hour at room temperature. Reaction mixture was partitioned between water (100 mL) and ethyl acetate (50 mL). The aqueous layer was extracted twice with 30 mL portions of ethyl acetate. The combined organic extracts were washed with water, brine, dried (Na₂SO₄), and concentrated in vacuo to provide brown oil. Its treatment by water provide yellow solid was filtered and purified by consecutive crystallization from ethanol to give 0.6 g of pure title compound as an yellow solid, m.p. 187-189°C.

| | | | | |
|---|---|---|---|---|
| Anal. Calcd. for C₁₄H₁₆N₄O₅S₂ | C, 43.74; | H, 4.20; | N, 14.57; | S, 16.68 |
| Found | C, 43.82; | H, 3.99; | N, 14.77; | S, 16.57 |

### Example 18

### 3,5-dinitro-2-tetramethylenedithiocarbamoylbenzamide (17)

A solution of 3,5-dinitro-2-chlorobenzamide (1.0 g, 4.0 mmol), sodium tetramethylenedithiocarbamate dihydrate (0.9 g, 4.4 mmol) and acetone (40 mL) was mixed for 0.5 hour at room temperature. Reaction mixture was diluted by water (80 mL), yellow solid was filtered and purified by crystallization from *i*-propanol to give 0.9 g of pure title compound as an light yellow crystalline solid, m.p. 116-118°C.

| | | | | |
|---|---|---|---|---|
| Anal. Calcd. for C₁₂H₁₂N₄O₅S₂ | C, 40.44; | H, 3.39; | N, 15.72; | S, 18.00 |
| Found | C, 40.57; | H, 3.42; | N, 15.64; | S, 17.87 |

### Example 19

### 3,5-dinitro-2-tetramethylenedithiocarbamoylpyridine (18)

A mixture of 3,5-dinitro-2-chloropyridine (0.5 g, 2.4 mmol), sodium tetramethylenedithiocarbamate dihydrate (0.6 g, 2.9 mmol) and ethanol (40 mL) was mixed for 3 hours at room temperature. Reaction mixture was diluted by water (80 mL), light tan yellow solid was filtered and purified by crystallization from mixture EtOH/DMF to give 0.5 g of pure title compound as an yellow crystalline solid, m.p. 157-159°C.

| | | | | |
|---|---|---|---|---|
| Anal. Calcd. for C₁₀H₁₀N₄O₄S₂ | C, 38.21; | H, 3.21; | N, 17.82; | S, 20.40 |
| Found | C, 38.03; | H, 3.22; | N, 17.79; | S, 20.44 |

### Example 20

### Determination of the inhibitory activity of the compounds of the invention against staphylococci and mycobacteria

The antibacterial activities of the compounds against multiresistent staphylococci (MRSA) strains 134/93 and 994/93 as well as against *Mycobacterium smegmatis* SG 987, *M. aureum* SB66, *M. vaccae* IMET 1010670 and *M. fortuitum* B were tested by determination of minimal inhibitory concentrations (MIC) by the micro broth dilution method in Mueller-Hinton broth (Difco) according to the NCCLS guidelines [National Committee for Clinical Laboratory Standards: Methods for dilution antimicrobial susceptibility tests for bacteria that grow aerobically; 4^{th} Ed.; Villanova, Ed.; Approved standard Document M7-A4. NCCLS, (1997)]
Activity against *M. tuberculosis* H37Rv was tested by the following method for determination of minimal inhibitory concentrations (MIC) and minimal bactericidal concentrations (MBC):

Strains were inoculated onto solid Lowenstein-Jensen medium. After 21 days, the cultures grown were used to prepare an inoculum suspension corresponding to 5 x 10⁸ microbial cells/ml). With 0,2 ml of that suspension tubes with 2 ml liquid Shkolnikova medium containing corresponding concentrations of compounds under study - from 100,0 to 0,195 µg/ml. were inoculated. After 14 days of incubation at 37 °C the tubes with liquid medium were centrifuged for 15min. at 3000 RPM. After discarding the supernatant, the sediment was resuspended in 0,8 ml of sterile 0,9% NaCl. 0,1 ml of the suspension was used to prepare smears subsequently stained by the Ziehl-Neelsen method. The remaining sediment was inoculated in 0,2 ml volumes into three tubes with solid drug free Lowenstein-Jensen medium to determine minimal bactericidal concentrations (MBC). The results were read after 21-28 days of cultivation at 37 °C. Controls were tubes cultured with test-strains not treated with the studied agents.

Minimal bactericidal concentration of drugs (MBC) was considered as the drug concentration completely inhibiting the growth of mycobacteria on the solid medium. The bacteriostatic effect (MIC) was characterized by the presence of only individual mycobacteria in the smear and a strong decrease in the number of colonies grown on solid media compared to the controls.

The results are presented in Tables 1 and 2.

**Table 1:**

| Antimicrobial activity of compounds as of the formula I determined by minimal inhibitory concentrations MIC [µg/ml] | | | | | | | | |
|---|---|---|---|---|---|---|---|---|
| Subst.Nr. | HKI-Nr. | 134/94 | 994/93 | SG 987 | SB 66 | 10670 | M.fort. | M.chel. |
| 1 | 9926004 | 3,12 | 25 | 12,5 | 25 | 3,12 | 25 | 12,5 |
| 2 | 9926075 | 1,56 | 3,12 | 6,25 | 12,5 | 3,12 | 6,25 | 3,12 |
| 3 | 9926088 | 3,12 | 6,25 | 12,5 | 12,5 | 3,12 | 6,25 | 3,12 |
| 4 | 9926097 | 0,78 | 1,56 | 6,25 | 6,24 | 1,56 | 6,25 | 3,12 |
| 5 | 9926101 | 1,56 | 3,12 | 6,25 | 12,5 | 3,12 | 6,25 | 3,12 |
| 6 | 9926108 | 3,12 | 3,12 | 12,5 | 12,5 | 3,12 | 12,5 | 12,5 |
| 7 | 10026016 | 1,56 | 3,12 | 12,5 | 6,25 | 3,12 | 6,25 | 12,5 |
| 8 | 10026023 | 3,12 | 6,52 | 12,5 | 6,25 | 3,12 | 12,5 | 12,5 |
| 9 | 10026068 | 0,4 | 0,4 | 3,12 | 1,56 | 3,12 | 6,25 | n.d. |
| 10 | 10026122 | 3,12 | 0,8 | 12,5 | 25 | 12,5 | 50 | n.d. |
| 11 | 10026125 | 0,8 | 0,8 | 6,25 | 1,56 | 0,8 | 1,56 | n.d. |
| 12 | 10026132 | n.d. | n.d. | 3,12 | 1,56 | 0,8 | 0,8 | n.d. |
| 13 | 10026137 | 3,12 | 1,56 | 3,12 | 3,12 | 1,56 | 1,56 | n.d. |
| 14 | 10126068 | 6,25 | 3,12 | 3,12 | 0,8 | 3,12 | 0,8 | n.d. |
| 15 | 10126069 | 3,12 | 3,12 | 3,12 | 1,56 | 0,8 | 1,56 | n.d. |
| 15a | 10126076 | 1,56 | 1,56 | 1,56 | 1,56 | 0,8 | 1,56 | n.d. |
| 16 | 10126078 | 6,25 | 12,5 | 6,25 | 6,25 | 0,1 | 0,8 | n.d. |
| 17 | 10126080 | 25 | 12,5 | 50 | 6,25 | 0,4 | 6,25 | n.d. |
| 18 | 10126095 | 1,56 | 12,5 | 6,25 | 3,12 | 3,12 | 3,12 | n.d. |
| n.d.: not determined | | | | | | | | |

**Table 2:**

| Antimicrobial activity of compounds of the formula I against *Mycobacterium tuberculosis* H37Rv as determined by minimal inhibitory concentrations (MIC) and minimal bactericidal concentrations (MBC) | | | |
|---|---|---|---|
| Subst.Nr. | HKI-Nr. | MIC (µg/ml) | MBC (µg/ml) |
| 1 | 9926004 | 31,3 | 100,0 |
| 2 | 9926075 | 20,8 | 29,1 |
| 3 | 9926088 | 5,37 | 7,29 |
| 4 | 9926097 | 6,25 | 12,5 |
| 5 | 9926101 | 9,38 | 12,5 |
| 6 | 9926108 | 20,8 | 41,7 |
| 7 | 10026016 | 7,78 | 10,42 |
| 8 | 10026023 | 7,81 | 12,5 |
| 9 | 10026068 | 4,78 | 6,25 |
| 10 | 10026122 | 5,21 | 8,33 |
| 11 | 10026125 | 9,37 | 12,5 |
| 12 | 10026132 | 3,64 | 8,33 |
| 13 | 10026137 | 3,12 | 6,25 |

## Claims

1. A compound of the formula I 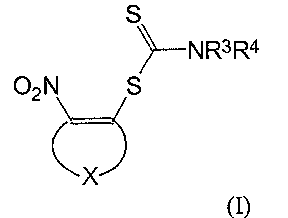 wherein X is a bivalent residue selected from the group consisting of 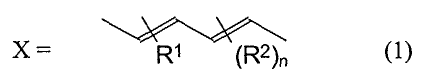 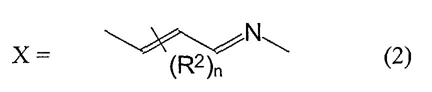 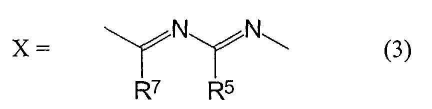 wherein
R¹ is CN, CONR³R⁴, CONHNR³R⁴;
R² is H, NO₂, CN, CONR³R⁴, COOR⁵, CHO, halogen or a saturated or unsaturated, linear or branched aliphatic radical having 1-7 chain members, OR⁵, SR⁵, NR³R⁴, trifluoromethyl, phenyl;
n is 0-3;
R³ and R⁴ are, independently from each other, H, a saturated or unsaturated, linear or branched aliphatic radical having 1-7 chain members, cycloalkyl having 3-6 carbon atoms, benzyl, naphthyl, OR⁵; or NR³R⁴ is morpholino; or R³ and R⁴ together represent a bivalent radical -(CH₂)ₘ- wherein m is 2-7, or a bivalent radical -(CH₂CH₂)NR⁶(CH₂CH₂)-, or R³ and R⁴ together represent a bivalent radical 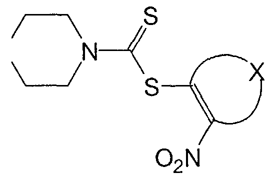 R⁵ is H or a saturated or unsaturated, linear or branched aliphatic radical having 1-7 chain members, trifluoromethyl, benzyl or phenyl;
R⁶ is H or a saturated or unsaturated, linear or branched aliphatic radical having 1-7 chain members, trifluoromethyl, benzyl or phenyl;
R⁷ is H, halogen or a saturated or unsaturated, linear or branched aliphatic radical having 1-7 chain members, OR⁵, SR⁵, NR³R⁴, trifluoromethyl, phenyl;
with the exclusion of
1,5-dinitro-3-cyano-6-dimethyldithiocarbamoyl-benzene, 3,5-dinitro-2-dimethyldithiocarbamoyl-pyridine, 3,5-dinitro-6-dimethyl-amino-2-dimethyl-dithiocarbamoyl-pyridine, 4-methoxy-6-diethyldithiocarbamoyl-5-nitro-pyrimidine, 4-methoxy-6-dipropyldithiocarbamoyl-5-nitro-pyrimidine, 4-dimethyl-amino-6-diethyldithiocarbamoyl-5-nitro-pyrimidine, 4-dimethylamino-6-dipropyldithio-carbamoyl-5-nitro-pyrimidine, and 4-methylamino-6-diethyldithiocarbamoyl-5-nitro-pyrimidine.

2. A 3,5-dinitro-2-R³R⁴-dithiocarbamoyl-benzonitrile of the formula (I) according to claim 1 wherein X is the residue of the formula (1), R¹ represents CN, R² is NO₂, n is 1 and R³ and R⁴ have the meanings given in claim 1.

3. A 3,5-dinitro-2-R³R⁴-dithiocarbamoyl-benzamide of the formula (I) according to claim 1 wherein X is the residue of the formula (1), R₁ represents CONR³R⁴, R² is NO₂, n is 1 and R³ and R⁴ have the meanings given in claim 1.

4. A 3,5-dinitro-2-R³R⁴-dithiocarbamoyl-pyridine of the formula (I) according to claim 1 wherein X is the residue of the formula (2), R² is NO_{2,} n is 1 and R³ and R⁴ have the meanings given in claim 1, except 3,5-dinitro-2-dimethyl-dithiocarbamoyl-pyridine.

5. A 4-R⁷-2-R⁵-6-R³R⁴-dithiocarbamoyl-5-nitro-pyrimidine of the formula (I) according to claim 1 wherein X is the residue of the formula (3), R³, R⁴, R⁵ and R⁷ have the meanings given in claim 1, except 4-methoxy-6-diethyldithio-carbamoyl-5-nitropyrimidine, 4-methoxy-6-dipropyldithiocarbamoyl-5-nitro-pyrimidine, 4-dimethylamino-6-diethyldithiocarbamoyl-5-nitro-pyrimidine, 4-dimethylamino-6-dipropyl-dithio-carbamoyl-5-nitro-pyrimidine and 4-methylamino-6-diethyldithiocarbamoyl-5-nitro-pyrimidine

6. A compound of the formula I according to claim 1 selected from the group consisting of
4-isopropylamino-6-diethyldithiocarbamoyl-5-nitropyrimidine,
4-cyclopropylamino-6-diethyldithiocarbamoyl-5-nitropyrimidine,
3,5-dinitro-2-diethyldithiocarbamoylbenzonitril,
4-methylhydroxyamino-6-diethyldithiocarbamoyl-5-nitropyrimidine,
4-ethoxy-2-methyl-6-diethyldithiocarbamoyl-5-nitropyrimidine,
4-methylamino-6-dimethyldithiocarbamoyl-5-nitropyrimidine,
4-dimethylamino-6-dimethyldithiocarbamoyl-5-nitropyrimidine,
3,5-dinitro-2-dimethyldithiocarbamoylbenzonitrile,
4-ethoxy-2-methyl-6-tetramethylenedithiocarbamoyl-5-nitropyrimidine,
4-propoxy-6-dimethyldithiocarbamoyl-5-nitropyrimidine,
4-methylamino-6-tetramethylenedithiocarbamoyl-5-nitropyrimidine,
4-dimethylamino-6-tetramethylenedithiocarbamoyl-5-nitropyrimidine,
4-methoxy-6-dimethyldithiocarbamoyl-5-nitropyrimidine,
4-methoxy-6-tetramethylenedithiocarbamoyl-5-nitropyrimidine,
3,5-dinitro-2-hexamethylenedithiocarbamoylbenzamide,
3,5-dinitro-2-tetramethylenedithiocarbamoylbenzamide,
3,5-dinitro-2-tetramethylenedithiocarbamoylpyridine,
3,5-dinitro-2-hexamethylenedithiocarbamoylbenzonitril, and
4-methylamino-6-heptamethylenedithiocarbamoyl-5-nitropyrimidine.

7. A pharmaceutical composition comprising a compound of the formula I according to claim 1.

8. A compound of the formula I 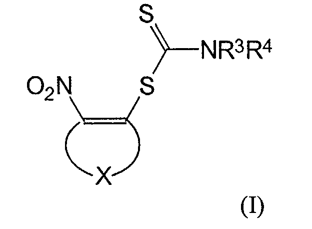 wherein X is a bivalent residue selected from the group consisting of 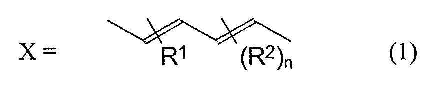 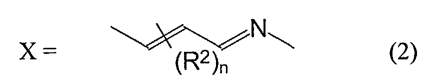 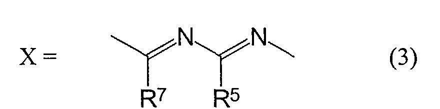 wherein
R¹ is CN, CONR³R⁴, CONHNR³R⁴;
R² is H, NO₂, CN, CONR³R⁴, COOR⁵, CHO, halogen or a saturated or unsaturated, linear or branched aliphatic radical having 1-7 chain members, OR⁵, SR⁵, NR³R⁴, trifluoromethyl, phenyl;
n is 0-3;
R³ and R⁴ are, independently from each other, H, a saturated or unsaturated, linear or branched aliphatic radical having 1-7 chain members, cycloalkyl having 3-6 carbon atoms, benzyl, naphthyl, OR⁵; or NR³R⁴ is morpholino; or R³ and R⁴ together represent a bivalent radical -(CH₂)ₘ- wherein m is 2-7, or a bivalent radical -(CH₂CH₂)NR⁶(CH₂CH₂)-, or R³ and R⁴ together represent a bivalent radical 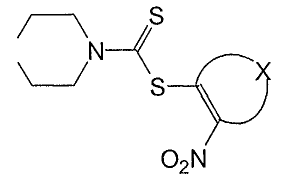 R⁵ is H or a saturated or unsaturated, linear or branched aliphatic radical having 1-7 chain members, trifluoromethyl, benzyl or phenyl;
R⁶ is H or a saturated or unsaturated, linear or branched aliphatic radical having 1-7 chain members, trifluoromethyl, benzyl or phenyl;
R⁷ is H, halogen or a saturated or unsaturated, linear or branched aliphatic radical having 1-7 chain members, OR⁵, SR⁵, NR³R⁴, trifluoromethyl, phenyl; for use in a method for the therapeutic or prophylactic treatment of bacterial infections in mammals.
